# EUROPEAN PATENT SPECIFICATION

(11) **EP 0 542 949 B1**
(45) Date of publication and mention of the grant of the patent: **04.03.1998**
(21) Application number: 92910019.6
(22) Date of filing: 08.05.1992
(51) Int. Cl.: A61K 39/29, C12N 7/02, C12P 21/08, G01N 33/576

(54) **HEPATITIS A VIRUS STRAIN, METHOD FOR THE ISOLATION OF SAID VIRUS STRAINS AND HEPATITIS A VACCINES**
HEPATITIS A VIRUSSTAMM, VERFAHREN ZUR ISOLIERUNG DIESES STAMMES UND HEPATITIS A VAKZINE
SOUCHE DU VIRUS DE L'HEPATITE A, PROCEDE D'ISOLATION DE CES SOUCHES ET VACCINS CONTRE L'HEPATITE A

(30) Priority: 08.05.1991 EP 91107526
(43) Date of publication of application: 26.05.1993
(73) Proprietor: SCHWEIZ. SERUM- & IMPFINSTITUT BERN, CH-3001 Bern (CH)
(72) Inventor: GLÜCK, Reinhard, CH-3028 Spiegal/Bern (CH); BRANTSCHEN, Stefan, CH-3013 Bern (CH)
(74) Representative: VOSSIUS & PARTNER
(86) International application number: EP9201013
(87) International publication number: WO9219268

(56) References cited:
- EP-A- 0 008 559
- EP-A- 0 025 745
- FR-A- 2 398 504
- Virus Genes 10:1,37-43,1995
- J. Clin. Invest.90,2491-2495,1992
- British Medical Bulletin 46:2,319-328,319-328

## Description

The invention relates to hepatitis A viruses (HAVs) having a serotype displaying the immunological characteristics of the HAV strain RG-SB XA112 (CNCM I-1080). In particular, the invention relates to the new hepatitis A virus strain RG-SB XA112 (CNCM I-1080). The invention also relates to structural components of said HAVs. Furthermore, the invention relates to processes for the isolation of said HAVs. The HAVs of the present invention and the structural components thereof can be used for the production of vaccines and diagnostic compositions. Finally, the invention relates to polyclonal and monoclonal antibodies which are directed to said new HAVs.

Hepatitis A infection is endemic in most less developed areas of the world, with widespread contamination of food and water supplies presenting a special risk to travellers from more developed areas. In developed countries, outbreaks associated with day care centers, handling diapered children and with drug abuse and homosexual activity are being increasingly recognized. Hepatitis A virus (HAV), classified as an enterovirus within the picornavirus family, is difficult to isolate in cell culture, grows poorly in early in vitro passages and generally is not cytopathogenic. The genome of the virus has been cloned and sequenced and only one serotype has been identified. Although infection with HAV never becomes chronic, it is the cause of significant human morbidity and loss of productivity.

For these reasons, there will be a future need for prophylaxis against hepatitis A virus. Passive immune prophylaxis is effective but of only temporary benefit and active immunization would be a more practical approach to the control of the infection.

Considerable progress has been made in the development of hepatitis A vaccines. Conventional inactivated and live-attenuated vaccines have been prepared from HAV replicated in cell culture. Recombinant DNA technology has been applied to develop alternative approaches to the development of hepatitis A vaccines. These approaches include expression of viral antigens in vitro, the use of live virus vectors for expression of viral antigens in vivo and the production of synthetic peptides representing gene products of the virus (Bulletin of the WHO, 66 (4) (1988), 443).

Since the important epitope for neutralization of the virus appears to be conformational rather than linear, further research will be necessary before hepatitis A vaccines based upon these new technologies become available. The feasibility of developing a conventional formaldehyde-inactivated HAV vaccine was demonstrated before the era of successful growth of HAV strain CR 326 in vitro, which was partially purified from the liver of acutely infected S. Labiatus marmosets (Provist, P.J. and Hilleman, M.R., Proc. Soc. Exp. Biol. Med.; 159 (1978), 201). Multiple aqueous doses of vaccine were administered to S. Labiatus marmosets. All animals immunized in this manner developed antibodies, and all were immune to challenge infection. Subsequently, a highly purified, formaldehyde-inactivated HAV vaccine was prepared from the same strain grown in LLC-MK2 cell culture, a transformed Rhesus monkey kidney cell line. This vaccine, however, is currently not acceptable for human vaccine development (Provist, P.J. et al. J. Med. Virol., 19 (1986), 23). Other prototype formaldehyde-inactivated HAV vaccines were prepared in other transformed cell lines or by passing through primary animal cell cultures: Merck & Co., Inc., (Provost, P.J., Hilleman, M.R. and Hughes, J.), US-A 5,021,348, US-A 4,301,249 and US-A 4,614,793, U.S. Dept. of Health and Human Services (Robertson), US-A 5,268,292, Seelig, R. et al., EP-A-0105066, Behringwerke AG (Lorenz), EP A-0 007461, U.S.A. (Daemer et al.) US-A 4,620,978.

Vaccines produced in such a manner have the following drawbacks:
(1) foreign antigens and genetic material from the growth substrate capable of inducing an allergic or oncogenic reaction may be contained in the vaccine;
(2) unknown agents originating from the passages through animal cell cultures may be contained in the vaccine;
(3) antibiotics contained in the vaccine may induce allergic reactions in sensitized individuals;
(4) the safety and immunogenicity of such HAV vaccines have not been sufficiently demonstrated in humans. An important advance in inactivated hepatitis A vaccine development has been the propagation of HAV in human fibroblast cultures without previous passaging in animals (Flehmig, B., EP 82-108268). The HAV was isolated from the stool of a patient with acute hepatitis A infection and propagated in primary human kidney cells. Further passages in human diploid fibroblast cells yielded the HAV antigen for the production of an inactivated hepatitis A vaccine. The vaccine produced in such a manner still has the following drawbacks:
   (I) isolation of HAV from stools and further cultivation on primary human cells without chemical pretreatment of the isolate still contains a potential risk of contamination with unknown extraneous agents,
   (II) serial passaging of HAV in human fibroblast cell culture yields large quantities of defective interfering particles (DI) (Calein and Roux, L., J. Virology, 62/8 (1988), 2859), which reduce the immunological potential of such vaccines,
   (III) the above mentioned DI suppress high titer harvests of HAV: therefore a vaccine produced in such a manner would be extremely expensive,
   (IV) the described HAV antigen is inactivated with a formaldehyde solution at a final dilution of 1:2000 in H₂O and stirred for 12 days at 37°C. This procedure denatures the HAV antigen so that the protective potential of the vaccine may be lost, and
   (V) the antigen developed in this manner didn't show a protective antibody titer against all hepatitis A isolates tested from all continents of the world.

Thus, the technical problem underlying the present invention is to provide an optimally tolerated hepatitis A vaccine capable of eliciting a high antibody titer and having superior immunological properties.

The solution to this technical problem is achieved by providing the embodiments characterized in the claims. In particular, it is achieved by providing HAVs having a serotype displaying the immunological characteristics of the HAV strain RG-SB XA112 (CNCM I-1080) which was deposited under the requirements of the Budapest Treaty at the Collection Nationale de Cultures de Microorganismes (CNCM) of the Institut Pasteur, Paris, on April 11, 1991, under the deposition number I-1080.

In a preferred embodiment the invention relates to the new HAV strain RG-SB XA112 (CNCM I-1080).

The above HAV displays superior immunogenic properties and furthermore induces a broad scope of protection against HAV infections. This is because the immune response induced by the new HAV strain is mediated by antibodies which also recognize most of the presently known other HAV serosubtypes.

The HAV of the present invention and the corresponding vaccines differ from the prior art in that
1st: The virus isolated from the stool of a patient with an acute hepatitis A infection has been purified by different physical purification methods and treated with an acid solution of pH 1 to remove all possible extraneous agents originating from the patient;
2nd: The virus has directly been passaged on human diploid cells of a controlled cell bank (a controlled cell bank is a bank wherein the cells have been extensively tested for the absence of extraneous contaminations or abnormalities) for vaccine production without passaging on primary animal or human cells, which reduces further contamination by these cells. In this context, the term "primary animal or human cells" refers to cells which are freshly isolated from animals or humans. They are not uniform in their nature and passaging of these cells is not possible. Therefore, an extensive testing procedure for the detection of abnormalities or contaminations cannot be carried out with these cells;
3rd: The passaging of the virus on human diploid cells has been performed in such a manner that the occurrence of DI has been avoided; and
4th: The viral antigen has been carefully treated by chemical agents to avoid denaturation of the proteins.

The present invention furthermore relates to structural components of the HAV of the present invention. Preferably, these structural components are the viral mRNA, the core protein, or the VP (Viral Protein) 1, VP2, VP3 or VP4 protein. The invention furthermore relates to biologically active or functional parts or derivatives of said structural components. A biologically active part of such structural components is for instance a part of VP1 which still induces the formation of anti-HAV antibodies and neutralizes active virus.

The structural components of the HAV of the present invention are involved in causing the immunological characteristics of the HAV strain RG-SB XA112 (CNCM I-1080) of the present invention.

In another embodiment, the present invention relates to a process for the isolation of HAV viruses comprising the following steps:
(a) Suspending stool of an acute phase HAV infected patient in a buffer solution;
(b) centrifugation of the suspension;
(c) ultracentrifugation of the supernatant;
(d) isolation of the virus containing fraction and dialysis thereof;
(e) infection of human diploid cells from a controlled cell bank of finite life cells with the virus preparation of (d);
(f) cultivation of the infected cells;
(g) passaging the cells of step (f) and assaying the passages for HAV activity;
(h) isolation of a virus containing cell extract of HAV positive passages of step (g); and
(i) further passaging and subsequent cloning of HAV strains by endpoint dilution, preferably after each third passage,
wherein said virus containing suspension or fraction is treated with an acid having a pH of lower than 2, preferably of 1, before infecting said human diploid cells.

It is known that only the enteroviruses show stable infectivity at pH 1. All other viruses are inactivated at this low pH. In comparison to other enteroviruses, HAV exhibits an extremely high stability at this low pH. We could show that from all human enteroviruses only HAV was still infectious after 5 hours of incubation at 37°C and pH 1. The employed procedure permits the conclusion that a contamination of the isolate with another human extraneous infectious agent can be excluded.

In a preferred embodiment of the process of the present invention, the cultivation and passaging up to step (g) is carried out at 37°C and the further passaging and cloning of step (i) is carried out at 32°C. The "cold adaptation process" to 32°C in human diploid cells leads to an attenuated HAV which is much less pathogenic in humans than a virus adapted at 37°C.

The term "attenuated HAV" in this context refers to an HAV whose pathogenicity is reduced.

The host cells for the virus multiplication are human diploid finite life cells, preferably MRC-5 (available from the American Type Culture Collection (ATCC) under deposition number ATCC CCL 171), MRC-9 (ATCC CCL 212) or WISTAR 38 cells. The term "human diploid finite life cells" refers to cells which undergo about 60 cell cycles in culture before they die. The advantage of the use of these cells lies in their safety as substrates. The finite life cells used underly extensive control concerning the absence of adventitious microbial agents including retroviruses, chromosomal abnormalities or oncogenic potential. In addition, it is evident that cells with a uniform population doubling (PD) level and prepared from a cell bank (depositary) give more reproducible results than primary cells from different individuals.

In another embodiment, the present invention relates to an HAV vaccine containing a non-pathogenic, immunogenic derivative of an HAV of the present invention, the HAV strain RG-SB XA112 (CNCM I-1080) of the present invention, and/or an immunogenic structural component of any of the HAVs of the present invention, and optionally a pharmaceutically acceptable carrier, adjuvant and/or diluent.

Besides the use of intact whole, inactivated HAV, a single or a combination of the four subunit capsid proteins VP1, VP2, VP3 and VP4 can be used for vaccine preparation. The whole virus or the subunit components can be adsorbed to known carrier materials such as aluminum hydroxide, aluminum phosphate, other viruses, liposomes, virosomes or immunosomes to enhance immunogenicity.

In a preferred embodiment of the HAV vaccine of the present invention, said derivative of said HAV is a chemically attenuated HAV or a non-pathogenic HAV strain which is additionally chemically attenuated.

A vaccine containing a chemically attenuated HAV refers to a vaccine which contains a precisely determined quantity of active virus in addition to the inactivated virus. It is thus a mixture of attenuated and inactivated types of vaccine as it has been described for rabies vaccine type "Fermi" (Lépine, P.: Laboratory Techniques in Rabies (WHO), 3rd edition, 1973, 199). Such a vaccine can be produced by limiting the formaldehyde concentration (e.g. 1:4000) and limiting the inactivation time and temperature (e.g. 4 days at 4°C). The advantage of such a vaccine lies in the higher immunogenicity: The inactivated particles stimulate the human immune system immediately after administration to human beings whereas the attenuated, cold adapted live HAV needs some time to replicate in the body before eliciting an immune response. Since the immune cells are only stimulated by the large amount of inactivated virus, but not by the small live virus fraction until it replicates, the live virus fraction infects its target cells, undergoes replication and after replication acts as an effective booster dose.

In another preferred embodiment of the HAV vaccine of the present invention, at least one of the HAV strains contained therein is HAV strain RG-SB XA112 (CNCM I-1080).

In a preferred embodiment of the present invention, the viruses contained in said vaccines have been chemically attenuated by formaldehyde or β-propiolactone (BPL) treatment. This principle of chemical attenuation can also be employed for the preparation of other viral vaccines such as containing the influenza virus, respiratory syncytial virus, or rotavirus.

Formaldehyde is known to chemically influence the capsid proteins in such a way that the virus is no longer able to be infectious without losing its immunogenicity. Instead of formaldehyde, β-propiolactone (BPL) can also be used as an inactivating agent.

In a preferred embodiment, the present invention relates to monoclonal antibodies which are specifically directed to the the HAVs of the present invention or to a structural component thereof. These antibodies of the present invention do not display any cross-reactivity with any other HAV serosubtypes known from the prior art. Thus, the provision of the new HAVs of the present invention for the first time allows the production of such specific monoclonal antibodies.

The monoclonal antibodies can be produced by immunizing human volunteers with a hepatitis A vaccine containing an inactivated, chemically attenuated or live HAV of the invention, e.g. strain RG-SB XA112 (CNCM I-1080), or a structural component thereof. Two weeks after successful immunization the stimulated lymphocytes can be isolated from the blood and fused with cells of a human myeloma cell line. The hybridomas thus obtained which synthesize the desired monoclonal antibody, can be selected by testing the cell culture medium containing the fused cells for the presence of said desired monoclonal antibody.

Finally, the present invention relates to a diagnostic composition containing the HAVs of the present invention or a non-pathogenic derivative thereof, HAV strain RG-SB XA112 (CNCM I-1080) or a non-pathogenic derivative thereof, a structural component of any of the HAVs of the present invention, or an antibody as set forth above.
The HAV antigen can e.g. be used to coat ELISA plates to detect HAV antibodies in the serum of a patient. The antibodies can e.g. be used to prepare conjugates for radioimmuno assays.

### EXAMPLE 1

### Isolation of HAV strain RG-SB from infectious material

Wild type HAV was obtained from stool of a patient in the acute phase of hepatitis A infection by suspension of the stool in a phosphate buffer solution, pH 7.4, centrifugation, pelleting the virus in the supernatant by ultracentrifugation through a sucrose cushion of 30% saccharose and further purified by density gradient ultracentrifugation using a CsCl₂ gradient with a density between 1.1 and 1.5 g/ml. HAV containing fractions were identified by a modified solid phase RIA technique. This modification refers to the determination of antigen instead of antibody. Said determination can be achieved by carrying out a so-called competition test: A constant quantity of anti-HAV antibodies is mixed with the HAV antigen. The amount of unbound antibodies which is then determined is a measure of the quantity of HAV antigen that neutralized the bound anti-HAV antibody portion.

The fraction with a density of about 1.3 g/ml was dialyzed twice against a physiological saline solution. The pH of the solution was adjusted with HCl (1 m) to pH 1. After 5 hours at room temperature the pH was adjusted with NaOH (10%) to pH 7.

### EXAMPLE 2

### Adaptation of HAV to human diploid cells (MRC-5)

Purified wild-type virus was adapted to MRC-5 (ATCC CCL 171) cells by mixing 1 ml of the isolated HAV material from Example 1 with 10 ml of a suspension containing BME medium (Gibco) and 10⁷ MRC-5 cells. The process of adaption enables the virus to replicate in said MRC-5 cells. This suspension was kept at room temperature and gently stirred every 10 minutes. After one hour, the suspension was transferred to a Corning tissue flask with a surface of 150 cm² and 70 ml of BME with 10% FBS was added. This was followed by adding CO₂ to the gas phase in the tissue flask to a final concentration of 5% and incubation at 37°C. The HAV-infected MRC-5 cells were split every week at a ratio of 1:2. One half of these cells was used for the adaptation process, the other cells for the testing for HAV antigen. After 10 blind passages (i.e. passages without the detection of HAV), HAV antigen could be detected for the first time using the modified RIA. For this purpose, the cell-associated virus was extracted by freezing-thawing the content of the Corning flasks three times and by subsequent ultrasonication. Cell debris was removed by low speed centrifugation.

For further attentuation the HAV of the 13th blind passage was used. The virus was isolated after breaking up the cells by freezing-thawing.

### EXAMPLE 3

### Attenuation of HAV strain RG-SB on MRC-5 cells

Confluent MRC-5 cell cultures grown in 75 cm² plastic flasks (Corning) were washed twice with BME medium and inoculated with 1.0 ml of viral inoculum containing the adapted HAV. After a four-hour period of viral adsorption at 32°C, BME medium containing 10% FBS was added. The cultures were incubated at 32°C and the medium was replaced thereafter at seven-day intervals. After each passage, the virus was extracted and passed onto a new freshly confluent tissue culture. The second passage lasted 4 weeks, the third one 3 weeks and the fourth one 2 weeks. After each passage, the virus was extracted from the lysate of the cells by freezing-thawing. After the fourth passage, several inocula were prepared for the fifth passage making endpoint dilutions of 10⁻⁵ to 10⁻⁹. The virus from the culture with the highest dilution in which virus could be detected was then used for further passaging. Passages were performed by incubating the virus-adsorbed cells at 32°C for 2 weeks (the medium was changed after one week) and by employing an endpoint dilution passage after each two passages. This procedure was repeated until the 23rd passage which yielded the adapted and attenuated production seed virus (i.e. the virus from this passage was used for the production of the vaccine) for hepatitis A vaccine.

### EXAMPLE 4

### Production of a live attenuated hepatitis A vaccine

Large quantities of MRC-5 cell cultures were grown in NUNC cell factories (NUNC, Copenhagen, Denmark). Dense cell layers were infected with HAV strain RG-SB XA112 (CNCM I-1080) from the production seed with a multiplicity of infection of about 0.1. The virus was adsorbed for 4 hours at 32°C. Fresh BME medium containing 10% FBS was then added to the infected cells and thereafter the cell factories were incubated at 32°C. After one week of incubation, the medium was replaced with fresh medium. After another week of incubation, the HAV was extracted from the cell culture. An extraction solution containing 100 mM borate and 5 mM Na-EDTA, adjusted to pH 8 with 10% NaOH, was added to the cultures. Subsequently, the NUNC cell factory was moved to a deep-freezing chamber at -30°C. After complete freezing of the cells, the cell factory was transferred to an incubation room of 37°C until the suspension had thawed. This procedure was repeated twice. The suspension was then pumped into centrifugation bottles and treated with ultrasonication (Bransonic, Branson Europe BV, frequency 50 kHz ±10%). Ten seconds of ultrasonic shocks repeated twice, after intervals of 10 seconds each, yielded the best result.

The suspension was then centrifuged for 10 minutes at 2500 xg and the supernatant was transferred into a bottle. The pellet was resuspended with half the volume of the extraction solution and freeze-thawed again, followed by ultrasonication and centrifugation. This procedure was repeated once and all the supernatants were finally pooled. Then the supernatants were clarified by filtration and sterile filtered through a membrane filter of pore size 0.2 µm (Millipore).

These supernatants containing the active pharmaceutical ingredient were stored at -30°C until the vaccine was blended.

The final bulk vaccine was prepared under sterile conditions and contained the following components:

| | |
|---|---|
| - Attenuated HAV virus, strain RG-SB XA112 (CNCM I-1080) | 10^{7.3}TCID₅₀/ml |
| - NaCl | 3.4 mg/ml |
| - Polygeline® or Physiogel® (Hausamman) | 16.0 mg/ml |
| - Phenolred (Sigma) | 20 µg/ml |
| - Sucrose | 340 µg/ml |

### EXAMPLE 5

### Production of an inactivated hepatitis A vaccine

HAV virus, strain RG-SB XA112 (CNCM I-1080), was prepared according to Example 4. The filtered supernatants were further purified by:
- Concentration by ultrafiltration (Minitan, Millipore);
- purification by ultracentrifugation through a 30% sucrose cushion solution (24 hours, 100'000 xg);
- resuspension of the pellet in 5 mM Na-EDTA, 100 mM borate, pH 8;
- further purification by ultracentrifugation through a CsCl₂ gradient with a density between 1.1 and 1.5 g/ml at 100'000 xg;
- pooling of fractions containing HAV (d < 1.35 - d > 1.23);
- transfer of the fractions into a dialysis tubing (Spectra, Por, Molecular cut off 12'000 - 14'000) and dialysis against 0.9% NaCl at 4°C, 3 x 12 hours;
- inactivation of the HAV suspension with formaldehyde (1:2000 dilution in H₂O), 3.5 days at 37°C and 6 days at room temperature;
- elimination of formaldehyde by dialysis or ultracentrifugation (see above);
- dilution of the inactivated HAV suspension with a 0.9% NaCl solution to an antigen concentration of 1000 ng/ml;
- adsorption of the antigen to an adjuvant (e.g. Al(OH)₃ or liposomes) by mixing equal amounts of the HAV suspension and the adjuvant solution (in the case of Al(OH)₃ a stock solution of 0.8% was used).
- Filling of 0.5 ml aliquots into vaccine vials.

### EXAMPLE 6

### Production of a partially inactivated hepatitis A vaccine (chemically attenuated)

The vaccine was prepared according to Example 5 with the following modification:

Instead of complete inactivation the purified HAV suspension was chemically attenuated by treatment with a formaldehyde solution (diluted 1:2000 in H₂O). The suspension was stirred at 4°C for 4 days. Formalin was removed by pelleting the virus twice by ultracentrifugation (100'000 xg, 1 hour). The HAV was resuspended in a 0.9% NaCl solution and then diluted to an antigen concentration of 100 ng per vaccine dose.

### EXAMPLE 7

### Vaccination of human volunteers with vaccines containing the HAV strain RG-SB XA112 (CNCM I-1080)

Three hepatitis A vaccines were prepared according to Examples 4, 5 and 6. All vaccines met the standards of international control authorities for inactivated and live attenuated vaccines produced in human diploid cells.

15 healthy seronegative adults were immunized orally with 10⁵ tissue culture infectious dose 50% (TCID₅₀) of vaccine produced as described in Example 4 (Vaccine A). At a TCID₅₀, 50% of all cells in the culture are infected when the virus stock solution is diluted by a factor of 10⁵.

15 healthy seronegative adults were immunized parenterally with an inactivated hepatitis A vaccine containing 250 ng of inactivated HAV antigen as described in Example 5 (Vaccine B).

Another group of 15 healthy seronegative adults was immunized parenterally with a chemically attenuated hepatitis A vaccine containing 100 ng of inactivated HAV antigen and 10⁵ TCID₅₀ of HAV produced according to Example 6 (Vaccine C).

Vaccine A was administered once, vaccines B and C were administered according to the following schedule: Two vaccinations on day O and a booster dose on day 7. Antibody titers of all volunteers were monitored on day 28 for vaccine A and on day 21 for vaccines B and C. Anti-HAV antibodies were determined with a commercially available RIA-kit (Abbott). The results are shown in Table 1.

**Table 1**

| Vaccine Group | Antibody titer in serum Geometric mean value (mIU) | Seroconversion n = 15 % |
|---|---|---|
| A | 769 | 100 |
| B | 359 | 100 |
| C | 1231 | 100 |

The results indicate that all HAV preparations containing strain RG-SB XA112 (CNCM I-1080) were highly immunogenic. All vaccines showed a significant seroconversion after vaccination.

In addition, all three vaccines were well tolerated: No systemic and only a few local reactions were reported after vaccination. In no group could enhanced liver enzyme values be detected.

### EXAMPLE 8

### Neutralization of different HAV strains with serum from volunteers vaccinated with the HAV strain RG-SB XA112 (CNCM I-1080)

Neutralization tests employing eight strains of HAV recovered from widely diverse geographic areas were carried out on serum specimens obtained from human volunteers after vaccination with the HAV strain RG-SB XA112 (CNCM I-1080). The serum from each volunteer neutralized HAV from Kansas (LV-374), Alaska (FrAL), Germany (Gr8), Panama (PA21), North Africa (MBB), Australia (HM-175), Switzerland (CLF) and Shanghai (Shanghai). These results indicate that the vaccine will protect against HAV from different parts of the world.

### EXAMPLE 9

### Production of polyclonal antibodies directed to HAV strain RG-SB XA112 (CNCM I-1080)

In order to obtain a potent polyclonal serum for use as a diagnostic tool for HAV antigen determination or anti-HAV antibody measurement, adult spf sheep were immunized with a vaccine prepared according to Example 5 on days 0, 7, 14 and 44. On day 0, four doses were administered i.m. to each sheep at different sites (thighs). On days 7, 14 and 44, two doses were injected i.m. into both hindlegs. On day 58, 350 ml of blood was collected from each sheep. The serum fraction was separated and further purified according to known techniques (Cohn, E.J. et al., J. Amer. Chem. Soc., 69 (1946), 459.).

### EXAMPLE 10

### Production of human monoclonal antibodies (HMAB) directed to HAV strain RG-SB XA112 (CNCM I-1080)

Adult volunteers received two intramuscular injections of the vaccine prepared according to Example 4 on day 0 and a booster dose on day 7 in the deltoid region. Human peripheral blood lymphocytes (PBL) were obtained from donors on day 28. Mononuclear cells were separated on a Ficoll®-Hypaque density gradient (Pharmacia, Uppsala, Sweden) and monocytes were depleted by adherence to plastic. Non-adherent cells were then tested in antigen-specific panning assays as follows: The cells were centrifuged and resuspended in cold PBS (pH 7.4) containing 1% BSA. Lymphocytes (10⁷/ml in PBS containing 1% BSA; PBS/BSA solution) were added to each well of a 6-well plate (Costar, Badhoevedorp, Netherlands), previously coated with HAV antigen. To block the remaining unspecific binding sites on the plastic surface, the wells had been incubated with the PBS/BSA solution for at least 1 hour at 4°C. After incubation for 70 minutes, the non-adherent cells were aspirated. IMDM cell culture medium (Sigma Chemical Co., St. Louis, MO) containing 10% fetal calf serum (FCS) and an equal volume of Epstein-Barr virus (EBV containing supernatant from a culture of the B 95-8 marmoset cell line) were added to the adherent cells. The cells were cultured at 37°C for 3 hours. After incubation, the cells were washed and distributed into 96 well microtiter plates containing IMDM plus 10% FCS, 30% conditioned medium from the lymphoblastoid cell line JW5, 5 x 10⁻⁵ M 2-mercaptoethanol, 50 µg/ml gentamycin sulfate (Sigma), and 600 ng/ml cyclosporine A (Sandimmun, Sandoz, Basel, Switzerland) at a density of 10⁴ to 10⁵ cells/well. After 14 to 21 days of incubation, culture supernatants were screened by ELISA for antibody binding to HAV, strain RG-SB XA112 (CNCM I-1080). Cell cultures form positive wells were expanded, retested by ELISA, subcultured at low density and further tested by immunoblotting and immunofluoroescence. After further expansion, the cells were fused with the 6-thioguanine/ouabain resistant F3B6 (NS1 x human B cell hybrid) heteromyeloma cell line by the plate fusion technique (Larrick, J.W., Human Hybridomas and Monoclonal Antibodies, Plenum Press, New York, 1985, p. 446). Hybrids were selected in IMDM containing 100 µM hypocanthine, 5 µg/ml azaserine and 5 µM ouabain and cultured in microtiter plates without a feeder layer. Hybridomas with supernatants containing antibodies which specifically bound HAV RG-SB XA112 (CNCM I-1080) (neutralization tests) were cloned by limiting dilution and HMAb secreted by the cells were harvested.

## Claims

1. Hepatitis A virus (HAV) having a serosubtype displaying the immunological characteristics of the HAV strain RG-SB XA112 (CNCM I-1080).

2. Hepatitis A virus strain RG-SB XA112 (CNCM I-1080).

3. A structural component of the HAV or strain according to claim 1 or 2, which is involved in causing the immunological characteristics of said HAV serosubtype or strain.

4. The structural component according to claim 3 which is the viral mRNA, core protein, or the VP1, VP2, VP3, or VP4 protein, or a biologically active or functional part or derivative thereof.

5. A process for the isolation of the HAV according to claim 1 or 2 comprising the following steps:
(a) suspending stool of an acute phase HAV infected patient in a buffer solution;
(b) centrifugation of the suspension;
(c) ultracentrifugation of the supernatant;
(d) isolation of the virus containing fraction and dialysis thereof;
(e) infection of human diploid cells from a controlled cell bank of finite life cells with the virus preparation of (d);
(f) cultivation of the infected cells;
(g) passaging the cells of step (f) and assaying the passages for HAV activity;
(h) isolation of a virus containing cell extract of HAV positive passages of step (g); and
(i) further passaging and subsequent cloning of HAV strains by endpoint dilution;
wherein said virus containing suspension or fraction is treated with an acid having a pH of lower than 2, preferably of 1, before infecting said human diploid cells.

6. The process according to claim 5 wherein the cultivation and passaging up to step (g) is carried out at 37°C and the further passaging and cloning of step (i) is carried out at 32°C.

7. The process according to claim 5 or 6 wherein said human diploid finite life cells are MRC-5, MRC-9 or WISTAR 38 cells.

8. An HAV vaccine containing a non-pathogenic, immunogenic derivative of an HAV according to claim 1, the HAV strain of claim 2, and/or an immunogenic structural component thereof according to claim 3 or 4 and optionally a pharmaceutically acceptable carrier, adjuvant, and/or diluent.

9. The HAV vaccine according to claim 8 wherein said derivative of said HAV is a chemically attenuated HAV.

10. The HAV vaccine according to claim 8 wherein the HAV strain is additionally chemically attenuated.

11. The vaccine according to claim 9 or 10 wherein said chemical attenuation results from a formaldehyde or β-propiolactone (BPL) treatment.

12. The vaccine according to claim 9, wherein said derivative of said HAV strain is inactivated.

13. The vaccine according to claim 9, wherein said HAV strain is inactivated.

14. A monoclonal antibody which is specifically directed to an HAV of claim 1 or 2 or to a structural HAV component of claim 3 or 4.

15. A diagnostic composition containing an HAV according to claim 1 or a derivative thereof, the HAV according to claim 2 or a derivative thereof, a structural HAV component according to claim 3 or 4 or an antibody according to claim 14.

16. A process for the preparation of a structural component according to claim 3 or 4 which comprises using standard techniques for the isolation of mRNA and proteins.

17. A process for the preparation of a vaccine according to any one of claims 8 to 13, wherein the HAV of claim 1 or 2 or the structural component of claim 3 or 4 is combined with a pharmaceutically acceptable carrier.

18. A process for the preparation of a monoclonal antibody according to claim 14 comprising the following steps:
(a) immunizing a human with a hepatitis A vaccine prepared according to claim 17;
(b) isolating the stimulated lymphocytes from the blood;
(c) fusing said lymphocytes with a human myeloma cell line;
(d) selecting hybridomas obtained by testing the cell culture medium;
(e) recovering the desired monoclonal antibodies from the culture medium.

19. A process for the preparation of a diagnostic composition according to claim 15, wherein the immunogenic compound of any one of claims 1 to 4 or the monoclonal antibody prepared according to claim 18 is combined with an appropriate carrier.

## Patentansprüche

1. Hepatitis A Virus (HAV) mit einem Serosubtyp, der die immunologischen Eigenschaften des HAV-Stamms RG-SB XA112 (CNCM I-1080) aufweist.

2. Hepatitis A Virusstamm RG-SB XA112 (CNCM I-1080).

3. Struktureller Bestandteil des HAV oder des Stamms nach Anspruch 1 oder 2, der die immunologischen Eigenschaften des HAV-Serosubtyps oder des Stamms mit verursacht.

4. Struktureller Bestandteil nach Anspruch 3, der die virale mRNA, das Kernprotein, das VP1, VP2, VP3, VP4 Protein oder ein biologisch aktiver oder funktioneller Teil oder Abkömmling davon ist.

5. Verfahren zum Isolieren des HAV nach Anspruch 1 oder 2, umfassend die folgenden Schritte:
(a) Suspendieren des Stuhls eines akut HAV-infizierten Patienten in einer Pufferlösung;
(b) Zentrifugieren der Suspension;
(c) Ultrazentrifugieren des Überstands;
(d) Isolieren der das Virus enthaltenden Fraktion und deren Dialyse;
(e) Infizieren von menschlichen diploiden Zellen aus einer kontrollierten Zellbank mit der Virusaufarbeitung aus (d);
(f) Kultivieren der infizierten Zellen;
(g) Passage der Zellen aus Schritt (f) und Testen der Passagen auf HAV-Aktivität;
(h) Isolieren eines Virus enthaltenden Zellextrakts aus HAV-positiven Passagen aus Schritt (g); und
(i) weitere Passage und anschließendes Clonieren von HAV-Stämmen mittels Endpunkt-Verdünnung;
wobei die Virus enthaltende Suspension oder Fraktion mit einer einen pH-Wert von weniger als 2, vorzugsweise 1, aufweisenden Säure behandelt wird, bevor die menschlichen diploiden Zellen infiziert werden.

6. Verfahren nach Anspruch 5, wobei das Kultivieren und die Passage bis zu Schritt (g) bei 37°C und die weitere Passage und das Clonieren von Schritt (i) bei 32°C ausgeführt wird.

7. Verfahren nach Anspruch 5 oder 6, wobei die Wirtszellen menschliche, diploide, begrenzt lebende Zellen, vorzugsweise MRC-5, MRC-9 oder WISTAR 38 Zellen sind.

8. HAV-Impfstoff, enthaltend einen nicht-pathogenen, immunogenen Abkömmling eines HAV nach Anspruch 1, des HAV-Stamms nach Anspruch 2 und/oder eines immunogenen strukturellen Bestandteils davon nach einem der Ansprüche 3 oder 4, gegebenenfalls in Verbindung mit einem pharmazeutisch verträglichen Träger, Adjuvans und/oder Verdünnungsmittel.

9. HAV-Impfstoff nach Anspruch 8, wobei der Abkömmling des HAV ein chemisch attenuiertes HAV ist.

10. HAV-Impfstoff nach Anpruch 8, wobei der HAV-Stamm zusätzlich chemisch attenuiert ist.

11. Impfstoff nach Anspruch 9 oder 10, wobei die chemische Attenuierung mittels Formaldehyd oder β-Propiolacton(BPL)-Behandlung erfolgt ist.

12. Impfstoff nach Anspruch 9, wobei der Abkömmling des HAV-Stamms inaktiviert ist.

13. Impfstoff nach Anspruch 9, wobei der HAV-Stamm inaktiviert ist.

14. Monoclonaler Antikörper, der spezifisch auf ein HAV nach Anspruch 1 oder 2 oder einen strukturellen HAV-Bestandteil nach Anspruch 3 oder 4 gerichtet ist.

15. Diagnostische Zusammensetzung, enthaltend ein HAV nach Anspruch 1 oder einen Abkömmling davon, das HAV nach Anspruch 2 oder einen Abkömmling davon, einen strukturellen HAV Bestandteil nach Anspruch 3 oder 4 oder einen Antikörper nach Anspruch 14.

16. Verfahren zur Herstellung eines strukturellen Bestandteils nach Anspruch 3 oder 4 umfassend die Verwendung von Standardtechniken wie Isolation von mRNA und Proteinen.

17. Verfahren zur Herstellung eines Impfstoffs nach einem der Ansprüche 8 bis 13, wobei das HAV nach Anspruch 1 oder 2 oder der strukturelle Bestandteil nach Anspruch 3 oder 4 mit einem pharmazeutisch verträglichen Träger kombiniert wird.

18. Verfahren zur Herstellung eines monoclonalen Antikörpers nach Anspruch 14, umfassend folgende Schritte:
(a) Immunisierung eines Menschen mit einem nach Anspruch 17 hergestellten Hepatitis A Impfstoff;
(b) Isolieren der stimulierten Lymphozyten aus dem Blut;
(c) Fusion der Lymphozyten mit einer menschlichen Myeloma-Zellinie;
(d) Selektion von Hybridomen die durch Testen des Zellkulturmediums erhalten werden; und
(e) Gewinnung der gewünschten monoclonalen Antikörper aus dem Kulturmedium.

19. Verfahren zur Herstellung einer diagnostischen Zusammensetzung nach Anspruch 15, wobei die immunogenen Bestandteile nach einem der Ansprüche 1 bis 4 oder der nach Anspruch 18 hergestellte monoclonale Antikörper mit einem geeigneten Träger kombiniert wird.

## Revendications

1. Virus de l'hépatite A (VHA) ayant un sous-type sérologique présentant les caractéristiques immunologiques de la souche de VHA RG-SB XA112 (CNCM I-1080).

2. Souche RG-SB XA112 (CNCM I-1080) du virus de l'hépatite A.

3. Composant structurel du VHA ou de la souche selon la revendication 1 ou 2, lequel est impliqué dans l'induction des caractéristiques immunologiques desdits sous-type sérologique ou souche de VHA.

4. Composant structurel selon la revendication 3, lequel est l'ARNm viral, une protéine de partie centrale ou la protéine VP1, VP2, VP3 ou VP4 ou une partie ou un dérivé biologiquement actifs ou fonctionnels de ceux-ci.

5. Procédé d'isolement du VHA selon la revendication 1 ou 2, comprenant les étapes suivantes :
(a) mise en suspension des selles d'un malade infecté par le VHA à la phase aiguë dans une solution tampon ;
(b) centrifugation de la suspension ;
(c) ultracentrifugation du surnageant ;
(d) isolement et dialyse de la fraction contenant le virus ;
(e) infection de cellules diploïdes humaines provenant d'une banque cellulaire contrôlée de cellules à durée de vie finie par la préparation virale de (d) ;
(f) culture des cellules infectées ;
(g) mise en oeuvre de passages des cellules de l'étape (f) et dosage de l'activité VHA des passages ;
(h) isolement d'un extrait cellulaire des passages positifs à VHA de l'étape (g) contenant le virus ; et
(i) mise en oeuvre de passages supplémentaires et clonage subséquent de souches de VHA par dilution à point final ;
où ladite suspension ou fraction contenant le virus est traitée par un acide ayant un pH inférieur à 2, de préférence égal à 1, avant l'infection desdites cellules diploïdes humaines.

6. Procédé selon la revendication 5, où la culture et les passages jusqu'à l'étape (g) sont effectués à 37°C et où les passages supplémentaires et le clonage de l'étape (i) sont effectués à 32°C.

7. Procédé selon la revendication 5 ou 6, où lesdites cellules diploïdes humaines à durée de vie finie sont des cellules MRC-5, MRC-9 ou WISTAR 38.

8. Vaccin contre le VHA contenant un dérivé immunogène, non pathogène, d'un VHA selon la revendication 1, la souche de VHA selon la revendication 2 et/ou un composant structurel immunogène de ces derniers selon les revendications 3 ou 4 et facultativement un véhicule, adjuvant et/ou diluant pharmaceutiquement acceptables.

9. Vaccin contre le VHA selon la revendication 8, où ledit dérivé du VHA est un VHA atténué chimiquement.

10. Vaccin contre le VHA selon la revendication 8, où la souche de VHA est en outre atténuée chimiquement.

11. Vaccin selon la revendication 9 ou 10, où ladite atténuation chimique résulte d'un traitement par le formaldéhyde ou la β-propionolactone (BPL).

12. Vaccin selon la revendication 9, où ledit dérivé de ladite souche de VHA est inactivé.

13. Vaccin selon la revendication 9, où ladite souche de VHA est inactivée.

14. Anticorps monoclonal spécifiquement dirigé contre un VHA selon la revendication 1 ou 2 ou contre un composant structurel de VHA selon la revendication 3 ou 4.

15. Composition diagnostique contenant un VHA selon la revendication 1 ou un dérivé de ce dernier, le VHA selon la revendication 2 ou un dérivé de ce dernier, un composant structurel du VHA selon la revendication 3 ou 4 ou un anticorps selon la revendication 14.

16. Procédé de préparation d'un composant structurel selon la revendication 3 ou 4, lequel comprend le recours à des techniques standards pour l'isolement de l'ARNm et des protéines.

17. Procédé de préparation d'un vaccin selon l'une quelconque des revendications 8 à 13, où le VHA selon la revendication 1 ou 2 ou le composant structurel selon la revendication 3 ou 4 est associé à un véhicule pharmaceutiquement acceptable.

18. Procédé de préparation d'un anticorps monoclonal selon la revendication 14, comprenant les étapes suivantes :
(a) immunisation d'un humain par un vaccin contre l'hépatite A préparé selon la revendication 17 ;
(b) isolement des lymphocytes stimulés du sang ;
(c) fusion desdits lymphocytes avec une ligne cellulaire de myélome humain ;
(d) sélection d'hybridomes obtenus par dosage du milieu de culture cellulaire ;
(e) récupération des anticorps monoclonaux désirés dans le milieu de culture.

19. Procédé de préparation d'une composition diagnostique selon la revendication 15, où le composé immunogène selon l'une quelconque des revendications 1 à 4 ou l'anticorps monoclonal préparé selon la revendication 18 est associé à un véhicule approprié.
